# EUROPEAN PATENT APPLICATION

(11) **EP 4 667 456 A1**
(43) Date of publication of application: **24.12.2025**
(21) Application number: 24757265.4
(22) Date of filing: 15.02.2024
(51) Int. Cl.: C07D 207/48, A61K 31/40, A61P 1/04

(54) **MANUFACTURING METHOD FOR 4-METHOXYPYRROLE DERIVATIVES**

(30) Priority: 15.02.2023 KR 20230020155
(71) Applicant: Daewoong Pharmaceutical Co., Ltd., Hwaseong-si, Gyenoggi-do 18623 (KR)
(72) Inventor: SON, Jeong-Hyun, Yongin-si, Gyeonggi-do 17027 (KR); SHIN, Jeong-Taek, Yongin-si, Gyeonggi-do 16973 (KR); KIM, Mi Jung, Sejong 30130 (KR); KIM, Ji Duck, Hwaseong-si, Gyeonggi-do 18454 (KR); PARK, Joon Seok, Gyeonggi-do 17000 (KR)
(74) Representative: Germain Maureau
(86) International application number: PCT/KR2024/095174
(87) International publication number: WO 2024/172545

(57) **Abstract**

The present invention relates to a manufacturing method for 4-methoxypyrrole derivatives. The preparation method according to one embodiment of the present invention has advantages that the number of production days is reduced by half, the process efficiency and yield can be improved, and 4-methoxypyrrole derivatives can be usefully obtained for industrial mass production.

## Description

### [TECHNICAL FIELD]

### CROSS-REFERENCE TO RELATED APPLICATION(S)

This application claims the benefit of Korean Patent Application No. 10-2023-0020155 filed on February 15, 2023 in the Korean Intellectual Property Office, the disclosure of which is incorporated herein by reference in its entirety.

The present invention relates to a manufacturing method for 4-methoxypyrrole derivatives.

### [BACKGROUND ART]

Gastrointestinal tract ulcer, gastritis, and reflux esophagitis are generated while the balance between offensive factors (for example, gastric acid, Helicobacter bacteria pepsin, stress, alcohol and tobacco, etc.) and defensive factors (for example, gastric mucosa, bicarbonate, prostaglandin, the degree of blood supply, etc.) is destroyed. Therefore, a therapeutic agent for treating gastrointestinal damage such as gastrointestinal tract ulcer, gastritis and reflux esophagitis is divided into a drug for inhibiting the offensive factors and a drug for enhancing the defensive factors.

On the other hand, it is reported that gastrointestinal tract ulcer, gastritis, and reflux esophagitis occur ulcers even without secretion of gastric acid. Thus, as much as the offensive factor increases, a reduction in the defensive factor due to a pathological change of the gastric mucosa is thought to play an important role in the occurrence of gastric ulcers. Thus, in addition to drugs for inhibiting the offensive factors, drugs for enhancing the defensive factors are used for the treatment of gastrointestinal tract ulcer and gastritis. As the drugs for enhancing the defensive factors, mucosal protective drugs which are attached to the ulcer site to form a physicochemical membrane, and drugs that promote the synthesis and secretion of mucus have been known.

On the other hand, *Helicobacter pylori,* which is a bacteria present in the stomach, has been known to cause chronic gastritis, gastric ulcer, duodenal ulcer and the like, and a large number of patients with gastrointestinal damages are infected with *H. pylori.* Therefore, these patients must take antibiotics such as clarithromycin, amoxicillin, metronidazole, tetracycline, together with anti-ulcer agents such as a proton pump inhibitor, or an acid pump antagonist. Consequently, various side effects have been reported.

Therefore, there is a need to develop anti-ulcer drugs which inhibit the secretion of gastric acid (for example, proton pump inhibitory activity) and enhance defensive factors (for example, an increase in mucus secretion) and at the same time have eradication activity against *H. pylori.*

In this connection, Korean Patent No. 10-1613245 discloses that 4-methoxy pyrrole derivatives or pharmaceutically acceptable salts thereof have excellent anti-ulcer activity (i.e., proton pump inhibitory activity, etc.) and eradication activity against *H*. *pylori,* and thus can be effectively used in the prevention and treatment of gastrointestinal damage due to gastrointestinal tract ulcer, gastritis, reflux esophagitis or *Helicobacterpylori.*

Specifically, the above prior patent discloses the following compound as a type of 4-methoxypyrrole derivative.

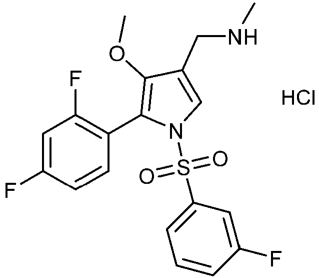

According to the description of the above prior patent, the preparation process for the compound consists of seven steps in total.

However, according to the preparation process of the above prior patent, the yield is as low as 4.63%, a high-temperature reaction is required, and expensive equipment is required, and thus, it is not suitable for industrial mass production.

Accordingly, Korean Patent No. 10-2233455 provided a manufacturing method for the following intermediates which is used in the preparation of 4-methoxypyrrole derivatives.

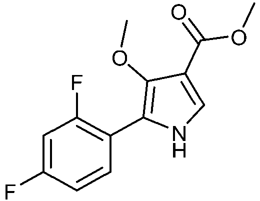

According to the description of the above prior patent, the preparation process of the intermediate compound consists of six steps in total.

In addition, Korean Patent No. 10-2126576 provided a manufacturing method for 4-methoxypyrrole derivatives from the above intermediate. According to the description of the prior patent, the preparation process of the intermediate compound consists of four steps in total.

However, when the 4-methoxypyrrole derivative is manufactured by combining the preparation processes of the two prior patents above, the total yield is as low as 20.2% and the preparation process consists of ten steps in total, and thus, is still not suitable for industrial mass production.

Therefore, the present inventors have found that the preparation process consists of six steps in total to reach the 4-methoxypyrrole derivative, and the compound is obtained in higher yield compared to the above-mentioned prior patents, and completed the invention.

### [DETAILED DESCRIPTION OF THE INVENTION]

### [Technical Problem]

It is an object of the present invention to provide a manufacturing method for 4-methoxypyrrole derivatives.

### [Technical Solution]

In order to achieve the above object, provided herein is a manufacturing method as shown in the following Reaction Scheme 1:

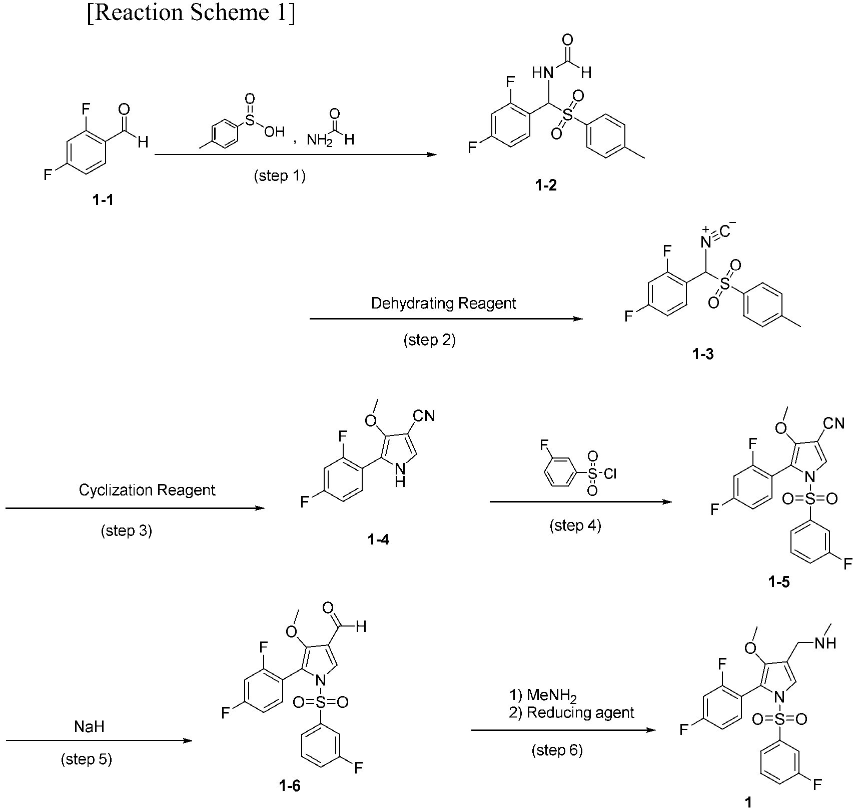

Specifically, in one embodiment of the present invention, there is provided a manufacturing method for the compound represented by Chemical Formula 1, comprising the following steps 1 to 6:
1) reacting the compound represented by Chemical Formula 1-1 with p-toluenesulfinic acid and formamide in the presence of an acid catalyst to prepare the compound represented by Chemical Formula 1-2;
2) reacting the compound represented by Chemical Formula 1-2 with a dehydrating reagent to prepare the compound represented by Chemical Formula 1-3;
3) reacting the compound represented by Chemical Formula 1-3 with a cyclization reagent to prepare the compound represented by Chemical Formula 1-4;
4) reacting the compound represented by Chemical Formula 1-4 with 3-fluorobenzenesulfonyl chloride to prepare the compound represented by Chemical Formula 1-5;
5) reacting the compound represented by Chemical Formula 1-5 with sodium hydride to prepare the compound represented by Chemical Formula 1-6; and
6) reacting the compound represented by Chemical Formula 1-6 with methylamine to form an intermediate, and then adding a reducing agent to convert the intermediate to the compound represented by Chemical Formula 1.

In the Korean Patent No. 10-2233455 and Korean Patent No. 10-2126576 discussed earlier, when preparing the compound represented by Chemical Formula 1 from the compound represented by Chemical Formula 1-1, many reaction steps must be performed, and the yield of the final material and the process efficiency are low.

For example, in order to prepare a compound represented by Chemical Formula 1 from a compound represented by Chemical Formula 1-1 by a combination of Korean Patent No. 10-2233455 and Korean Patent No. 10-2126576, a total of 10 reaction steps must be performed, which is a factor of decreasing the yield of the final material and the process efficiency.

On the other hand, the one embodiment has advantages that the reaction steps required are simplified, the process efficiency and yield of the final material are improved compared to the above prior patents, and the number of the production days is reduced by half, which makes it useful for industrial mass production of 4-methoxypyrrole derivatives.

Next, an embodiment of the present invention will be described in detail for each of the above steps. It is also possible to control the quality of the final material by adjusting the process temperature, performance time, etc. of each of the above steps with reference to the following explanation.

### (Step 1)

The step 1 is a step of reacting the compound represented by Chemical Formula 1-1 with p-toluenesulfinic acid and formamide in the presence of an acid catalyst to prepare the compound represented by Chemical Formula 1-2, which is a step of introducing a formamide group and a substituted phenylsulfonyl group into the compound represented by Chemical Formula 1-1.

First, the step 1 may include a step of preparing a p-toluenesulfinic acid (4-methylbenzenesulfinic acid) concentrate before reacting with the compound represented by Chemical Formula 1-1. Thereby, the concentrated p-toluenesulfinic acid can be used for the reaction between the compound represented by Chemical Formula 1-1 and formamide.

p-Toluenesulfinic acid concentrate may be prepared by mixing sodium p-toluenesulfinate with water and an organic solvent under acid conditions, separating the organic layer from the mixture, mixing the organic layer with sodium sulfate, removing and concentrating water. Here, the p-toluenesulfinic acid concentrate may be concentrated to 10 mol% or more, more specifically 50 to 500 mol%.

Preferably, the acid that can be used includes hydrochloric acid, nitric acid, sulfuric acid, or phosphoric acid, and preferably, hydrochloric acid is used.

Preferably, the organic solvent that can be used includes ethyl acetate, diethyl ether, dimethyl ether, diisopropyl ether, tert-butyl methyl ether, or a mixture of two or more thereof. Preferably, the organic solvent may be ethyl acetate.

Preferably, before mixing the sodium sulfate, the method may further comprise a step of separating the organic layer of the mixture of sodium p-toluenesulfinate, water, and ether, mixing the organic layer with a saturated sodium chloride solution, and then separating the organic layer again.

Preferably, the concentrating step may be a step of concentrating under reduced pressure.

Next, the concentrated residue of the prepared p-toluenesulfinic acid is mixed and reacted with a compound represented by Chemical Formula 1-1 (2,4-difluorobenzaldehyde) and formamide in the presence of an acid catalyst to prepare a compound represented by Chemical Formula 1-2.

Preferably, the molar ratio between the compound represented by Chemical Formula 1-1 and formamide may be 10:1 to 1:10, specifically 5:1 to 1:5.

Preferably, the reaction may be performed in the presence of chlorotrimethylsilane (TMSCl), camphorsulfonic acid, trifluoromethanesulfonic acid (TfOH), trifluoroacetic acid, benzoic acid, nitrobenzoic acid, hydrochloric acid, calcium chloride, or a mixture of two or more thereof as the acid catalyst. The above compounds can be used as an acid catalyst for the Mannich reaction, which is the reaction of the step 1. Preferably, the reaction may be performed in the presence of chlorotrimethylsilane, trifluoromethanesulfonic acid, or a mixture thereof.

Preferably, the molar ratio between the compound represented by Chemical Formula 1-1 and the acid catalyst may be 20:1 to 1:10, and more preferably 10:1 to 1:5.

Preferably, acetonitrile, tetrahydrofuran, methylene chloride, methanol, ethanol, propanol, iso-propanol, butanol, tert-butanol, toluene, or a mixture of two or more thereof may be used as the reaction solvent. Specifically, a mixture of toluene and acetonitrile can be used.

Preferably, the reaction is performed at 0°C to 80°C. Preferably, the reaction is performed at 10°C to 70°C or 20°C to 60°C. If the reaction temperature is too low, there is a problem that the yield of step 1 decreases, and if the reaction temperature is too high, side reactions may occur or only the manufacturing costs are borne without a substantial increase in manufacturing yield. Preferably, the reaction temperature may be the internal temperature of the reactor.

Preferably, the second reaction is performed for 1 hour to 36 hours. Preferably, the reaction is performed for 5 to 30 hours. If the reaction time is too short, there is a problem that the reaction does not proceed sufficiently and the manufacturing yield in step 1 decreases. If the reaction time is too long, there may be a problem that only the manufacturing costs are borne without a substantial increase in manufacturing yield.

After the reaction of the step 1 is completed, the method may further include a step of concentrating and purifying the compound represented by Chemical Formula 1-2, if necessary. Preferably, the purification may be performed by crystallizing the compound represented by Chemical Formula 1-2 from the reaction product of the step 1.

As the solvent for crystallizing the compound represented by Chemical Formula 1-2 from the reaction product of step 1, an alcohol compound alone or a mixed solvent with water can be used. Specific examples of the alcohols may include methanol, ethanol, propanol, butanol, isopropyl alcohol, or a mixture of two or more thereof. For example, the reaction product of step 1 can be crystallized by a process of adding methanol alone, isopropyl alcohol alone, or a mixture thereof with water in a temperature range of 0°C to 30°C, stirring the mixture for 10 minutes to 1 hour, and filtering it under reduced pressure, and then washing the filtrate. The filtrate can be washed with water or an alcohol compound, and specifically, can be washed with methanol, isopropyl alcohol, or a mixture thereof with water.

After purifying the compound represented by Chemical Formula 1-2, it can be dried at 40°C to 80°C for 12 to 48 hours to reduce the moisture content contained in the compound represented by Chemical Formula 1-2. Specifically, the drying may be vacuum drying.

More specifically, if the moisture content of the compound represented by Formula 1-2 is significantly lowered by drying at 40°C to 80°C, the conversion rate of the next step (i.e., step 2 below) can be increased.

### (Step 2)

The step 2 is a step of reacting the compound represented by Chemical Formula 1-2 with a dehydrating reagent to prepare a compound represented by Chemical Formula 1-3.

Preferably, the step 2 is a step of converting the formamide group present in the compound represented by Chemical Formula 1-2 to an isocyanide group to prepare a compound represented by Chemical Formula 1-3.

Preferably, the dehydrating reagent comprises phosphoryl chloride, trichloromethyl chloroformate, bis(trichloromethyl)carbonate, triphenylphosphine, thionyl chloride, p-toluenesulfonic acid or a mixture of two or more thereof. The dehydrating reagent can convert a formamide group to an isocyanide group.

Preferably, the molar ratio between the compound represented by Chemical Formula 1-2 and the dehydrating reagent is 20:1 to 1:10, and more preferably 10:1 to 1:5.

Preferably, the step 2 may be performed in the presence of a base. Preferably, the base may be trimethylamine, triethylamine, diisopropylamine, diisopropylethylamine, pyridine, potassium carbonate, potassium hydrogen carbonate, sodium carbonate, sodium hydrogen carbonate, sodium hydroxide, potassium hydroxide, lithium hydroxide, sodium methylate, potassium butyrate, or cesium carbonate, and triethylamine is preferred. Preferably, the molar ratio between the compound represented by Chemical Formula 1-2 and the base is 20:1 to 1:20, and more preferably 10:1 to 1:10.

Preferably, the reaction solvent of step 2 may include 1,2-dimethoxyethane, tetrahydrofuran, 2-methyltetrahydrofuran, dimethyl carbonate, acetonitrile, ethylacetate, N,N-dimethylformamide, carbon tetrachloride, methylene chloride or a mixture of two or more thereof. Specifically, 1,2-dimethoxyethane may be used as the reaction solvent.

Preferably, the step 2 is performed in a temperature range from -20°C to 20°C. Preferably, the reaction temperature may be -10°C to 10°C. If the reaction temperature is too low, there is a problem that the yield of the step 2 decreases, and if the reaction temperature is too high, there may be a problem that the yield decreases due to an increase in reaction by-products. Preferably, the reaction temperature may be the internal temperature of the reactor.

Preferably, the second reaction is performed for 10 minutes to 10 hours. Preferably, the reaction is performed for 30 minutes to 8 hours, or 1 hour to 4 hours. If the reaction time is too short, the reaction does not proceed sufficiently and the manufacturing yield of step 1 decreases, and if the reaction time is too long, there may be a problem that only the manufacturing costs are borne without a substantial increase in the manufacturing yield.

After the reaction of the step 2 is completed, the method may further comprise a step of purifying the compound represented by Chemical Formula 1-3, if necessary. Preferably, the purification may be performed by crystallizing the compound represented by Chemical Formula 1-3 from the reaction product of the step 2.

The solvent for crystallizing the compound represented by Chemical Formula 1-3 from the reaction product of the step 2 may include ethyl acetate, butyl acetate, isopropyl acetate, n-hexane, n-heptane, methanol, water, or a mixture of two or more thereof. For example, the reaction product of step 2 can be crystallized by a process of adding ethyl acetate to extract an organic layer, concentrating the organic layer, sequentially mixing methanol and water with the concentrated residue, stirring the mixture for 10 minutes to 1 hour, filtering it under reduced pressure and then washing the filtrate. In addition, sodium hydrogen carbonate can be used before crystallization and during washing of the organic layer for the purpose of completing the reaction and removing byproducts.

The compound represented by Chemical Formula 1-3 is purified, and then dried at 40°C to 80°C for 12 to 48 hours, so that the moisture content contained in the compound represented by Chemical Formula 1-3 can be lowered. Preferably, drying may be vacuum drying.

More specifically, if the moisture content of the compound represented by Chemical Formula 1-3 is significantly lowered by drying at 40°C to 60°C, the conversion rate in the next step (i.e., step 3 below) can be increased.

### (Step 3)

The step 3 is a step of reacting the compound represented by Chemical Formula 1-3 with a cyclization reagent to prepare the compound represented by Chemical Formula 1-4.

Specifically, the cyclization reagent includes ethyl (Z)-2-cyano-3-methoxyacrylate (CMA), methyl (Z)-2-cyano-3-methoxyacrylate, propyl (Z)-2-cyano-3-methoxyacrylate, or a mixture of two or more thereof.

Preferably, the molar ratio between the compound represented by Chemical Formula 1-3 and the cyclization reagent may be 10:1 to 1:10, and more preferably 5:1 to 1:5.

The reaction solvent of step 3 may include acetonitrile, tetrahydrofuran, methylene chloride, methanol, ethanol, propanol, iso-propanol, butanol, tert-butanol, toluene, or a mixture of two or more thereof. Specifically, methanol can be used.

The step 3 is preferably performed in the presence of a base. Specifically, the base may be trimethylamine, triethylamine, diisopropylamine, diisopropylethylamine, potassium carbonate, potassium hydrogen carbonate, sodium carbonate, sodium hydrogen carbonate, sodium hydroxide, lithium hydroxide, potassium hydroxide, sodium methylate, potassium butyrate, or cesium carbonate, and preferably, potassium carbonate is used. Preferably, the molar ratio between the compound represented by Chemical Formula 1-3 and the base is 20:1 to 1:10, and more preferably 10:1 to 1:5.

The reaction temperature in the step 3 may be 50°C to 150°C based on the external temperature of the reactor. Preferably, the reaction temperature may be 50°C to 100°C. If the reaction temperature is too low, there is a problem that the manufacturing yield decreases, and if the reaction temperature is too high, there may be a problem that the yield decreases due to an increase in reaction by-products.

Preferably, the second reaction is performed for 30 minutes to 10 hours. Preferably, the reaction is performed for 1 hour to 5 hours. If the reaction time is too short, the reaction does not proceed sufficiently, which causes a problem that the manufacturing yield of step 3 decreases, and if the reaction time is too long, there may be a problem that only the manufacturing costs are borne without a substantial increase in the manufacturing yield.

After the reaction of the step 3 is completed, the method may further comprise a step of purifying the compound represented by Chemical Formula 1-4, if necessary. Preferably, the purification may be performed by crystallizing the compound represented by Chemical Formula 1-4 from the reaction product of the step 3.

The solvent for crystallizing the compound represented by Chemical Formula 1-4 from the reaction product of the step 3 may include ethyl acetate, butyl acetate, isopropyl acetate, n-hexane, n-heptane, or a mixture of two or more thereof. For example, after the reaction in step 3 is completed, ethyl acetate and water are mixed, the organic layer is separated, and then the separated organic layer may be sequentially washed with water and sodium chloride solution, and concentrated under reduced pressure. Then, an organic solvent such as methanol can be added to the concentrated residue, purified water can be added, cooled, and then stirred. Crystallization can then be performed by filtering the crystals under reduced pressure and then washing the filtrate.

The compound represented by Chemical Formula 1-4 is purified, and then dried at 40°C to 80°C for 12 to 48 hours, so that the moisture content contained in the compound represented by Chemical Formula 1-4 can be lowered. Preferably, drying may be vacuum drying. More specifically, the drying temperature may be 40°C to 60°C.

### (Step 4)

The step 4 is a step of reacting the compound represented by Chemical Formula 1-4 with 3-fluorobenzenesulfonyl chloride to prepare the compound represented by Chemical Formula 1-5.

Before reacting the compound represented by Chemical Formula 1-4 with 3-fluorobenzenesulfonyl chloride, the method may further comprise a step of dissolving the compound in an organic solvent together with a catalyst to prepare a composition containing the compound represented by Chemical Formula 1-4.

The catalyst may be a nucleophilic catalyst. More specifically, the catalyst includes one or more nucleophilic catalysts selected from the group consisting of 4-dimethylaminopyridine(DMAP), 4-pyrrolidinylpyridine(PPY), pyridine(PY), 1,5,7-triazabicyclo[4.4.0]dec-5-ene(TBD), 1,8-diazabicyclo[5.4.0]undec-7-ene(DBU), and 7-methyl-1,5,7-triazabicyclo[4.4.0]dec-5-ene(MTBD). Preferably, the catalyst may be 4-dimethylaminopyridine (DMAP).

Preferably, the molar ratio between the compound represented by Chemical Formula 1-4 and the catalyst may be 100:1 to 1:10, specifically 20:1 to 1:3.

The organic solvent may be acetonitrile, tetrahydrofuran, methylene chloride, methanol, ethanol, propanol, iso-propanol, butanol, tert-butanol, toluene, or a mixture of two or more thereof. Specifically, acetonitrile can be used. Preferably, the solvent can be used in a volume amount (mL/g) that is 1 to 10 times the weight of the compound represented by Chemical Formula 1-4.

Subsequently, the compound represented by Chemical Formula 1-4 and 3-fluorobenzenesulfonyl chloride can be reacted under basic conditions.

The bases may include trimethylamine, triethylamine, diisopropylamine, diisopropylethylamine, potassium carbonate, potassium hydrogen carbonate, sodium carbonate, sodium hydrogen carbonate, sodium hydroxide, lithium hydroxide, potassium hydroxide, sodium methylate, potassium butyrate, or cesium carbonate. Preferably diisopropylethylamine is used.

Preferably, the molar ratio between the compound represented by Chemical Formula 1-4 and the base may be 10:1 to 1:10, more specifically 5:1 to 1:5.

In the step 4, the molar ratio between the compound represented by Chemical Formula 1-4 and 3-fluorobenzenesulfonyl chloride may be 10:1 to 1:10, more specifically 5:1 to 1:5.

The reaction temperature in step 4 may be 0°C to 60°C. Preferably, the reaction temperature may be 15°C to 55°C. If the reaction temperature is too low, there is a problem that the manufacturing yield decreases, and if the reaction temperature is too high, side reactions may occur, and only manufacturing costs are borne without a substantial increase in manufacturing yield.

Preferably, the step 4 is performed by stirring for 30 minutes to 10 hours. Preferably, it is performed for 1 hour to 5 hours. If the reaction time is too short, there is a problem that the reaction does not proceed sufficiently and the manufacturing yield in step 4 decreases, and if the reaction time is too long, there may be a problem that only the manufacturing costs are borne without a substantial increase in the manufacturing yield.

After the reaction in step 4 is completed, the method may further comprise a step of purifying the reaction product with the compound represented by Chemical Formula 1-5, if necessary. Preferably, the purification may be performed by crystallizing the compound represented by Chemical Formula 1-5 from the reaction product of the step 4.

Preferably, the reaction product of the step 4 is mixed with ethyl acetate and water, and then the organic layer is separated. Next, the separated organic layer is concentrated, mixed and concentrated with methanol, then methanol is added again, and the mixture is stirred at a temperature of 40°C to 60°C, cooled to room temperature, and then mixed with water. Crystallization can be performed by filtering the produced crystals under reduced pressure and then washing the filtrate.

The compound represented by Chemical Formula 1-5 is purified, and then dried at 40°C to 80°C for 12 to 48 hours, so that the moisture content contained in the compound represented by Chemical Formula 1-5 can be lowered.

More specifically, if the moisture content of the compound represented by Chemical Formula 1-5 is significantly lowered by drying at 40°C to 60°C, the conversion rate in the next step (i.e., step 5 below) can be increased.

### (Step 5)

The step 5 is a step of reacting the compound represented by Chemical Formula 1-5 with sodium hydride to prepare the compound represented by Chemical Formula 1-6.

Preferably, the step 5 is a step of converting the isocyanide group present in the compound represented by Chemical Formula 1-5 to an aldehyde group to prepare the compound represented by Chemical Formula 1-6.

Preferably, the molar ratio between the compound represented by Chemical Formula 1-5 and sodium hydride may be 20:1 to 1:10, and more preferably 10:1 to 1:5.

The sodium hydride may be used in the form of a dispersion in mineral oil. Specifically, a dispersion in liquid paraffin can be used. By using sodium hydride dispersed in mineral oil, the sodium hydride is in a dry solid state, minimizing contact with air and reducing the risk of fire.

Preferably, in the step 5, acetonitrile, tetrahydrofuran, methylene chloride, methanol, ethanol, propanol, iso-propanol, butanol, tert-butanol, toluene or a mixture of two or more thereof may be used as a reaction solvent, and specifically, tetrahydrofuran (THF) can be used as the solvent.

Preferably, the step 5 may be performed in the presence of zinc halide. The zinc halide may include zinc chloride, zinc bromide, zinc iodide, or a mixture of two or more thereof. The zinc halide plays the role of adjusting the reactivity of the reduction reaction with sodium hydride in the step 5 to reduce side reactions, and of assisting the production of the compound represented by Chemical Formula 1-6, and their presence can improve the reaction efficiency in step 5.

Here, the molar ratio between the compound represented by Chemical Formula 1-5 and zinc halide may be 10:1 to 1:10, more specifically 5:1 to 1:5.

The reaction temperature of step 5 may be 20°C to 80°C. Preferably, the reaction temperature may be 40°C to 60°C. If the reaction temperature is too low, there is a problem the manufacturing yield decreases, and if the reaction temperature is too high, there may be a problem that the yield decreases due to an increase in reaction by-products.

Preferably, the step 5 is performed by stirring for 1 hour to 36 hours. Preferably it is performed for 5 to 24 hours. If the reaction time is too short, there is a problem that the reaction does not proceed sufficiently and the manufacturing yield in step 5 decreases, and if the reaction time is too long, there may be a problem that only the manufacturing costs are borne without a substantial increase in the manufacturing yield.

Specifically, the compound represented by Chemical Formula 1-5 and the solvent are stirred within a reactor at room temperature, then the zinc chloride and sodium hydride are added, and the mixture is stirred at 40°C to 60°C for 5 to 24 hours. Thereby, the step 5 can be proceeded.

Hydrogen and heat may be generated due to the reaction between the compound represented by Chemical Formula 1-5 and the sodium hydride. Therefore, after the reaction in the step 5 is completed, the reactor is cooled until the internal temperature reaches -5°C to 5°C. For example, when cooling the reactor until the internal temperature reaches -5°C to 5°C, it is possible to improve safety on production scale.

After cooling the internal temperature of the reactor, the method may include a step of purifying the compound represented by Chemical Formula 1-6, if necessary. More specifically, the purification may be performed by crystallizing the compound represented by Chemical Formula 1-6 from the reaction product of the step 5.

Specifically, after cooling the reaction product of the step 5, ethyl acetate, butyl acetate, isopropyl acetate, or a mixture of two or more thereof are mixed with water, and then the pH is adjusted to 1.0 to 2.0 with an acid solution, and the organic layer can be separated. Then, sodium sulfate is mixed with the organic layer, followed by filtration and concentration under reduced pressure.

Subsequently, the concentrated residue is mixed with methanol, ethanol, propanol, butanol, isopropyl alcohol, ethyl acetate, butyl acetate, isopropyl acetate, n-hexane, n-heptane, or a mixture of two or more thereof alone, or a mixture thereof with water as a crystallization solvent, and then the mixture can be stirred for 10 minutes to 1 hour. Then, after filtration under reduced pressure, the filtrate is washed and vacuum-dried at 40°C to 80°C for 12 to 48 hours, thereby reducing the moisture content contained in the compound represented by Chemical Formula 1-6.

### (Step 6)

The step 6 is a step of reacting the compound represented by Chemical Formula 1-6 with methylamine to form an intermediate, and then adding a reducing agent to convert the intermediate to the compound represented by Chemical Formula 1.

Specifically, in the step 6, the compound represented by Chemical Formula 1-6 produces an imine compound through an imine production reaction with methylamine. Since such an imine compound corresponds to an intermediate with an unstable structure, it can be easily converted to the compound represented by Chemical Formula 1 through a reduction reaction.

The reductive imination reaction of step 6 may be performed in a reaction solvent, which is methanol, ethanol, isopropanol, dichloromethane, dichloroethane, tetrahydrofuran, ethyl acetate, dimethyl ether, acetonitrile or a mixture of two or more thereof.

More specifically, the compound represented by Chemical Formula 1-6 and methanol are added to a reactor separate from step 6, then cooled to 10°C to 15°C. Methylamine is then added thereof, and stirred at 10°C to 30°C for 20 minutes to 2 hours, whereby the compound represented by Chemical Formula 1-6 and the methylamine can react in a state sufficiently dissolved in the solvent to produce an imine compound.

At this time, the stirring time and temperature can be adjusted taking this into consideration that as the solubility of the compound represented by Chemical Formula 1-6 becomes lower, flexible materials contained in the final material may increase. For example, when stirred at 10°C to 15°C for 30 minutes to 1 hour, the compound represented by Chemical Formula 1-6 may be sufficiently dissolved, and if the reaction proceeds in this state, the content of flexible materials in the final material may decrease.

On the other hand, the reduction reaction of the intermediate (i.e., imine compound) produced by the reaction between the compound represented by Chemical Formula 1-6 and methylamine can proceed more stably at low temperatures.

In consideration of this point, after the reaction between the compound represented by Chemical Formula 1-6 and methylamine is completed, the reactor is cooled until the temperature reaches a range of -10°C to 0°C, for example, -10°C to -5°C, a reducing agent is added in accordance with the cooled temperature range, and the mixture can be stirred while maintaining the reactor temperature at -5°C to 10°C, for example, -5°C to 0°C. In such a low temperature range, the intermediate (i.e., imine compound) can stably react with the reducing agent and be converted to the compound represented by Chemical Formula 1.

Here, the reducing agent may include sodium borohydride, sodium cyanoborohydride, sodium triacetoxyborohydride, or a mixture of two or more thereof, the molar ratio between the compound represented by Chemical Formula 6 and the methylamine may be 10:1 to 1:10, and the molar ratio between the compound represented by Chemical Formula 6 and the reducing agent may be 10:1 to 1:10. Specifically, each molar ratio may be 5:1 to 1:5, more specifically 3:1 to 1:3.

After sufficiently reacting the intermediate (i.e., imine compound) with the reducing agent, an acid aqueous solution containing hydrochloric acid, glutamic acid, malonic acid, succinic acid, tartaric acid, oxalic acid, fumaric acid, phosphoric acid, methanesulfonic acid, or a mixture of two or more thereof may be supplied to adjust the pH in order to terminate the reaction (work-up). For example, the pH may be adjusted to 6.5 to 7.5, more specifically 7.0 to 7.5 by supplying an aqueous hydrochloric acid solution of 5 to 7 N.

Thereafter, extraction may be performed 1 to 3 times using an organic solvent to obtain an organic layer. the organic layer is washed with an aqueous base solution to remove acidic related substances, which are decomposition products generated during the process. A drying agent may be added thereto, stirred, and then filtered under reduced pressure. Then, the filtrate is washed, the pH of the filtrate was adjusted to 6.0 to 6.5, and the filtrate may be concentrated under reduced pressure. The pH of the filtrate is adjusted to 6.0 to 6.5 before concentration under reduced pressure, so that the efficiency of removing unknown related substances during crystallization is increased, and the product represented by Chemical Formula 1 can be obtained in high purity without any further purification steps.

During the extraction, an organic solvent such as ethyl acetate, diethyl ether, dimethyl ether, diisopropyl ether, methyl tertiary butyl ether, acetone, methyl ethyl ketone, methyl isobutyl ketone, or a mixture of two or more thereof may be used.

As a base used for preparing the base aqueous solution, potassium carbonate, potassium hydrogen carbonate, sodium carbonate, sodium hydrogen carbonate, sodium hydroxide, lithium hydroxide, potassium hydroxide, sodium methylate, potassium butyrate, or cesium carbonate may be used, and preferably, sodium hydrogen carbonate may be used.

Further, examples of the drying agent used after the extraction may include magnesium sulfate, sodium sulfate, and the like.

The acid or the mixed solution thereof with an organic solvent used to adjust the pH of the filtrate obtained after drying may be hydrochloric acid, glutamic acid, malonic acid, succinic acid, tartaric acid, oxalic acid, fumaric acid, phosphoric acid, methanesulfonic acid, or a mixture of two or more thereof; or a mixed solution thereof with an organic solvent. The organic solvent in the mixed solution may be selected from the organic solvents presented above. For example, a mixed solution in which ethyl acetate, diethyl ether, or a mixture thereof is used as the organic solvent, and hydrochloric acid is dissolved therein at a concentration of 0.5 to 2.0 M may be used to adjust the pH

### (Additional step - step of preparing acid addition salt)

The compound represented by Chemical Formula 1 may be in the form of a pharmaceutically acceptable salt. The salt includes conventional acid addition salts, for example, salts derived from inorganic acids such as hydrochloric acid, hydrobromic acid, sulfuric acid, sulfamic acid, phosphoric acid, or nitric acid, and salts derived from organic acids such as acetic acid, propionic acid, succinic acid, glycolic acid, stearic acid, maleic acid, hydroxy maleic acid, phenylacetic acid, glutamic acid, benzoic acid, salicylic acid, sulfanylic acid, 2-acetoxy-benzoic acid, fumaric acid, toluenesulfonic acid, methanedisulfonic acid, ethanedisulfonic acid, oxalic acid, or trifluoroacetic acid. Preferably, the salt may be hydrochloride or fumarate.

In order to supply the compound represented by Chemical Formula 1 in the form of a pharmaceutically acceptable salt, a step of adding an acid to the compound represented by Chemical Formula 1 to obtain a pharmaceutically acceptable acidic salt represented by Chemical Formula 1-1 may be further included after the step 6:

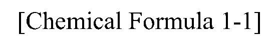

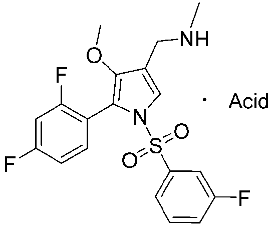

Specifically, the additional step may include a step of supplying an organic solvent to the compound represented by Chemical Formula 1, and then supplying an acid or a mixed solution thereof with an organic solvent to crystallize the acidic salt represented by Chemical Formula 1-1.

More specifically, an organic solvent may be supplied to the concentrated residue of step 6 containing the compound represented by Chemical Formula 1. The organic solvent supplied at this time may be ethyl acetate, diethyl ether, dimethyl ether, diisopropyl ether, methyl tertiary butyl ether, acetone, methyl ethyl ketone, methyl isobutyl ketone, methanol, ethanol, isopropyl alcohol, acetonitrile, dichloromethane, normal hexane, or a mixture of two or more of thereof. For example, it may be ethyl acetate.

After supplying the organic solvent to the concentrated residue of step 6 containing the compound represented by Chemical Formula 1, followed by stirring, the temperature inside the reactor is adjusted to 0°C to 5°C, and the acid or the mixed solution thereof with an organic solvent is supplied. Thereafter, the acidic salt represented by Chemical Formula 1-1 may be crystallized by stirring at 18°C to 22°C.

The acid or the mixed solution thereof with an organic solvent used to crystallize the acidic salt represented by Chemical Formula 1-1 may be hydrochloric acid, glutamic acid, malonic acid, succinic acid, tartaric acid, oxalic acid, fumaric acid, phosphoric acid, methanesulfonic acid, or a mixture of two or more thereof; or a mixed solution thereof with an organic solvent. The organic solvent in the mixed solution may be selected from the organic solvents presented above. For example, a mixed solution in which ethyl acetate, diethyl ether, or a mixture thereof is used as the organic solvent, and hydrochloric acid is dissolved therein at a concentration of 0.5 to 2.0 M may be used to crystallize the acidic salt represented by the Chemical Formula 1-1.

The temperature range of 18°C to 22°C for crystallization of the acidic salt represented by Chemical Formula 1-1 is determined in consideration of the fact that the solubility of impurities decreases and the purity of the acid salt represented by Chemical Formula 1-1 decreases when the reaction temperature is less than 18°C, and only manufacturing costs are borne without a substantial increase in manufacturing yield when the reaction temperature exceeds 22°C.

In addition, in order to crystallize the acidic salt represented by Chemical Formula 1-1, the acid or the mixed solution thereof with an organic solvent is supplied and then stirred for at least 1 hour. And, the stirring time may be controlled to 12 hours or less in order to prevent precipitation of related substances during stirring.

Compared to the existing technology, the present disclosure has the advantage that the same level of quality can be obtained even if the acid salt is crystallized only once.

Meanwhile, there is provided a pharmaceutical composition for the prevention or treatment of gastrointestinal damage due to gastrointestinal tract ulcers, gastritis, reflux esophagitis or Helicobacter pylori (H. pylori), comprising the compound represented by Chemical Formula 1 or a pharmaceutically acceptable salt thereof.

Also, there is provided a pharmaceutical composition for the prevention or treatment of 5-HT receptor-mediated or muscarinic acetylcholine receptor-mediated diseases comprising the compound represented by Chemical Formula 1 or a pharmaceutically acceptable salt thereof. In this case, the 5-HT receptor-mediated or muscarinic acetylcholine receptor-mediated diseases may be depression, manic depression, schizophrenia, autism, obsessive-compulsive neurosis, anxiety disorder, migraine, hypertension, eating disorder, irritable bowel syndrome (IBS), peptic ulcer, diabetic neuropathy, asthma, or overactive bladder.

The pharmaceutical composition may include a commonly used pharmaceutically acceptable carrier such as excipients, disintegrants, sweetening agents, lubricants or flavoring agents. The pharmaceutical composition may be formulated into preparations for oral administration such as tablets, capsules, powders, granules, suspensions, emulsions or syrups; or preparations for parenteral administration such as injections in accordance with conventional methods. The preparations can be formulated into various forms, for example, in a single dosage form or multiple dosage forms.

The pharmaceutical composition may be orally or non-orally administered. The non-oral administration may include, for example, intravenous, intramuscular, intraperitoneal, subcutaneous, rectal, and local administration. The composition may preferably be administered orally. Therefore, the composition may be formulated into various forms such as tablets, capsules, aqueous solutions or suspensions. In the case of tablets for oral administration, a carrier such as lactose or corn starch and a lubricant such as magnesium stearate may be commonly added. In the case of capsules for oral administration, lactose and/or dried corn starch may be used as a diluent. When an aqueous suspension is required for oral use, active ingredients may be combined with emulsions and/or suspensions. If necessary, certain sweetening and/or flavoring agents may be added. For intramuscular, intraperitoneal, subcutaneous and intravenous administration, sterile solutions of active ingredients are usually prepared, and the pH of the solutions should be suitably adjusted and buffered. For intravenous administration, the total concentration of solutes should be controlled in order to render the preparation isotonic. The composition according to the present disclosure may be in the form of an aqueous solution containing a pharmaceutically acceptable carrier such as saline with a pH of 7.4. The solution may be introduced into a patient's intramuscular blood-stream by local bolus injection.

In this case, the pharmaceutical composition may be administered in a therapeutically effective amount. Therefore, the compound represented by Chemical Formula 1 or a pharmaceutically acceptable salt thereof contained in the pharmaceutical composition may be administered in an effective amount ranging from about 0.01 mg/kg to about 100 mg/kg per day to a subject patient. Of course, the dosage may be changed according to the patient's age, weight, susceptibility, symptom or the efficacy of the compound.

### [Advantageous Effects]

As described above, according to one embodiment of the present invention, the process efficiency and yield are improved, which can be advantageous for industrial mass production of 4-methoxypyrrole derivatives.

### [DETAILED DESCRIPTION OF THE EMBODIMENTS]

Hereinafter, the present invention will be described in more detail by way of the following examples. However, the following examples are for illustrative purposes only, and the scope of the present invention is not limited thereto.

Analysis of the compounds prepared in the following Examples was performed as follows: Nuclear magnetic resonance (NMR) spectrum analysis was performed on a Bruker 400 MHz spectrometer, chemical shift was analyzed in ppm, and column chromatography was performed on silica gel (Merck, 70-230 mesh) (W. C. Still, J. Org. Chem., 1978 (43), 2923-2925).

### EXAMPLE

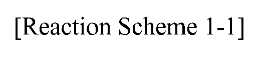

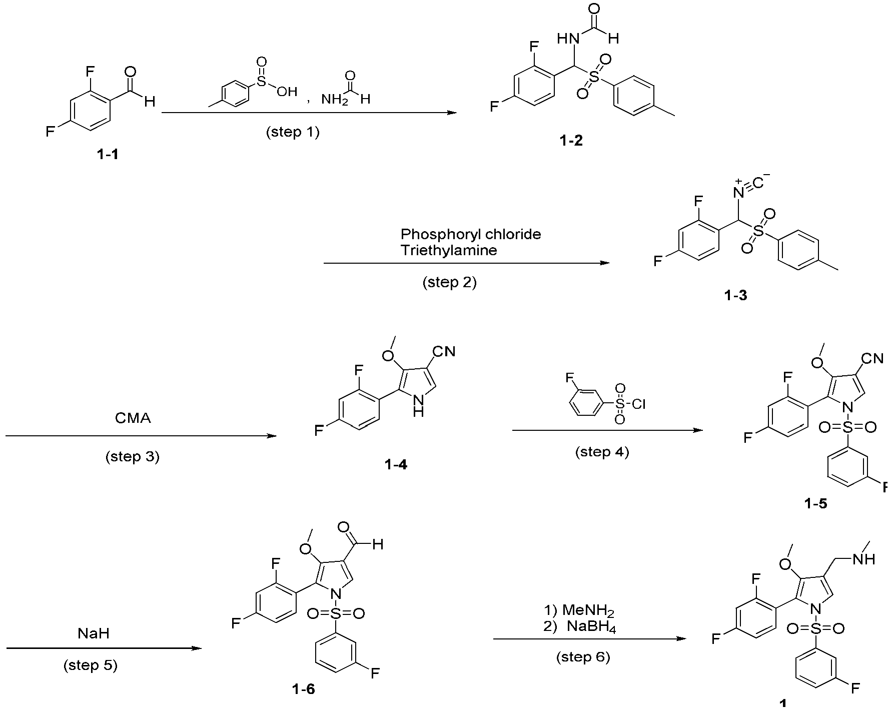

24.0 g of sodium p-toluenesulfinate, 100.0 mL of purified water, 100 mL of ethyl acetate, and 12.0 mL of hydrochloric acid were added to a flask, stirred for 10 minutes and allowed to stand for 10 minutes. Then, the aqueous layer was discarded and the organic layer was separated. 20.0 mL of saturated sodium chloride solution was then added to the organic layer, stirred for 10 minutes, allowed to stand for 10 minutes, and then the aqueous layer was discarded. 6.0 g of sodium sulfate was added to the organic layer, stirred for 5 minutes, filtered under reduced pressure, and concentrated under reduced pressure at 40°C to 50°C to prepare a p-toluenesulfinic acid concentrate concentrated to 150 mol%.

46.0 mL of toluene, 46.0 mL of acetonitrile, 12.2 g of the compound represented by Chemical Formula 1-1 (2,4-difluorobenzaldehyde), 9.9 g of formamide, and 10.2 g of chlorotrimethylsilane (TMSCl) were added to the flask containing the concentrated residue obtained from the above concentrate, and stirred at 30°C for 18 hours.

Then, the resulting mixture was concentrated at an external temperature of 40°C to remove the solvent. 73.2 mL of methanol was added to the concentrated residue, stirred at 50°C for 30 minutes, then cooled to room temperature, and further stirred for 1 hour. The resulting crystals were filtered under reduced pressure, and the filtrate was washed with 24.4 mL of methanol. The filtrate thus washed was put in a dryer, and then vacuum-dried at a temperature of 50°C to 60°C for 12 hours or more to obtain 25.8 g of the compound represented by Chemical Formula 1-2 (yield: 92.3%).
¹H NMR (500 MHz, DMSO): δ 9.93 (d, 1H), 8.02 (d, 1H), 7.73 (td, 1H), 7.67 (d, 2H), 7.45 (d, 2H), 7.39 - 7.33 (m, 1H), 7.29 (td, 1H), 6.51 (d, 1H), 2.42 (s, 3H)

### (Step 2)

10.1 g of the compound represented by Chemical Formula 1-2 and 60.0 mL of 1,2-dimethoxyethane were added to a flask, and the mixture was cooled to 0°C to 5°C, and stirred. 11.5 g of phosphoryl chloride was gradually added to the flask over 5 minutes.

Then, a mixed solution of 15.5 mL of 1,2-dimethoxyethane and 15.6 g of triethylamine was gradually added to the flask over 10 minutes. Then, the mixture was stirred for 1 hour while maintaining the temperature at -5°C to 0°C.

Then, 10.0 g of sodium hydrogen carbonate was dissolved in 150.0 mL of purified water, and then this solution was gradually added and stirred vigorously for 10 minutes. 150.0 mL of ethyl acetate was added to the stirred mixture, stirred for 5 minutes, and then allowed to stand. The aqueous layer was discarded and the organic layer was concentrated at an external temperature of 40°C. 50.0 mL of methanol was added to the obtained concentrated residue and stirred, then 50.0 mL of purified water was gradually added dropwise, stirred at room temperature for 20 minutes, and the resulting crystals were filtered under reduced pressure. The filtrate was put in a dryer, and vacuum-dried at a temperature of 50°C to 60°C for 12 hours or more to obtain 7.1 g of the compound represented by Chemical Formula 1-3 (yield: 74.4%).
¹H NMR (500 MHz, DMSO): δ 7.70 (d, 2H), 7.54 (d, 2H), 7.47 - 7.39 (m, 2H), 7.26 (td, 1H), 7.10 (s, 1H), 2.46 (s, 3H)

### (Step 3)

8.0 g of the compound represented by Chemical Formula 1-3, 4.0 g of ethyl (Z)-2-cyano-3-methoxyacrylate (CMA), 7.2 g of potassium carbonate (K₂CO₃) and 160.0 mL of methanol were added to a flask. The mixture was stirred at reflux for 2 hours at a reactor external temperature of 80°C.

The reaction solution was concentrated under reduced pressure at a temperature of 40°C to 45°C. 160.0 mL of ethyl acetate(EA) and 160.0 mL of purified water were added to the concentrated residue in the flask, and the mixture was stirred for 10 minutes, and allowed to stand for 10 minutes, and then the aqueous layer was discarded. The organic layer was washed with 80.0 mL of purified water and 40.0 mL of saturated sodium chloride solution. The organic layer was concentrated under reduced pressure at an external temperature of 40°C. 60.0 mL of methanol was added to the concentrated residue, stirred and dissolved. 30.0 mL of purified water was gradually added thereto and then cooled to 0 to 5°C and stirred for 1 hour.

The resulting crystals were filtered under reduced pressure, and washed with a mixed solution of 20.0 mL of methanol and 10.0 mL of purified water. The filtrate was put in a dryer, and vacuum-dried at a temperature of 50°C to 60°C for 12 hours or more to obtain 4.7 g of the compound represented by Chemical Formula 1-4 (yield: 76.8%).
¹H NMR (500 MHz, CDCl₃): δ 8.93 (s, 1H), 8.00 (td, 1H), 7.15 (d, 1H), 6.96 (dddd, 1H), 6.89 (ddd, 1H), 4.04 (s, 3H)

### (Step 4)

7.5 g of the compound represented by Chemical Formula 1-4, 0.8 g of 4-dimethylaminopyridine (DMAP), and 37.5 mL of acetonitrile were added to a flask, and the mixture weas stirred and dissolved.

7.5 g of 3-fluorobenzenesulfonyl chloride and 5.0 g of N,N-diisopropylethylamine (DIPEA) were added thereto, and stirred at 35°C to 40°C for 2 hours to complete the reaction.

Then, 37.5 mL of purified water and 37.5 mL of ethyl acetate(EA) were added thereto, stirred for 10 minutes, and allowed to stand for 10 minutes, and then the aqueous layer was discarded. After concentrating the organic layer, 15.0 mL of methanol was added to the concentrated residue, stirred at room temperature, dissolved and then concentrated again. 37.5 mL of methanol was added to the concentrated residue, stirred at 50°C to 60°C, dissolved, and then cooled to room temperature. 22.5 mL of purified water was added to a flask for 10 minutes, and stirred for 1 hour. The resulting crystals were filtered under reduced pressure, and the filtrate was washed with 15.0 mL of purified water. The filtrate thus washed was put in a dryer, and then vacuum-dried at a temperature of 50°C to 60°C for 12 hours or more to obtain 11.4 g of the compound represented by Chemical Formula 1-5 (yield: 90.7%).
¹H NMR (500 MHz, CDCl₃): δ 7.84 (s, 1H), 7.45 (td, 1H), 7.40 - 7.30 (m, 1H), 7.22 (dt, 1H), 7.18 (td, 1H), 7.04 (dt, 1H), 6.94 (td, 1H), 6.77 (td, 1H), 3.73 (s, 3H)

### (Step 5)

10.0 g of the compound represented by Chemical Formula 1-5 and 100.0 mL of tetrahydrofuran (THF) were added to a flask, and stirred at room temperature for 10 minutes.

5.2 g of zinc chloride and 3.1 g of sodium hydride (60%, dispersion in liquid paraffin) were added thereto, and stirred at a temperature of 45°C to 55°C for 15 hours.

The reaction solution was cooled to 0°C to 5°C, and 50.0 mL of purified water and 50.0 mL of ethyl acetate (EA) were added, and the mixture was stirred and adjusted to pH 1.0 to 2.0 with 6N-HCl solution, and then stirred for 10 minutes, allowed to stand for 10 minutes, and the aqueous layer was discarded. 10.0 g of sodium sulfate was added to the organic layer, stirred for 10 minutes, filtered under reduced pressure, and then concentrated. 40.0 mL of ethanol and 10.0 mL of water were added to the concentrated residue, and stirred at room temperature for 1 hour.

The resulting crystals were filtered under reduced pressure, and washed with 20.0 mL of a mixed solution of ethanol and water. The filtrate was put in a dryer, and vacuum-dried at a temperature of 40°C to 50°C for 12 hours or more to obtain 7.5 g of the compound represented by Chemical Formula 1-6 (yield: 74.4%).
¹H NMR (500 MHz, CDCl₃): δ 9.89 (s, 1H), 7.99 (s, 1H), 7.44 (td, 1H), 7.33 (tdd, 1H), 7.24 (dt, 1H), 7.18 (td, 1H), 7.06 (dt, 1H), 6.97 - 6.89 (m, 1H), 6.77 (td, 1H), 3.63 (s, 3H)

### (Step 6)

100.0 g of the compound represented by Chemical Formula 1-6 obtained in step 5 and 396.0 g of methanol were added to a flask, cooled to 10°C to 15°C, and then 42.7 g of methylamine was added thereto, and stirred at 10°C to 15°C for 1 hour.

Then, the internal temperature was cooled down to -10°C to -5°C. While maintaining the temperature range of -5°C to 0°C, 4.8 g of sodium borohydride was added in divided portions, and then stirred at -5°C to 0°C for 30 minutes to complete the reaction.

After completion of the reaction, 1,000 g of purified water was gradually added while maintaining the internal temperature at 10°C to 25°C, and then 902.0 g of ethyl acetate was added thereto. Then, the internal temperature was maintained at 10°C to 25°C, and the pH was adjusted to 7.0 to 7.5 using 6N-hydrochloric acid aqueous solution.

Then, the mixture was stirred for 10 minutes, and allowed to stand for 30 minutes to separate the layers, and the organic layer was stored. 451.0 g of ethyl acetate was added to the resulting aqueous layer, stirred for 10 minutes, and then allowed to stand for 10 minutes. Thereafter, layer separation was performed, and the organic layer was combined with the organic layer obtained earlier, and the same re-extraction process was performed again.

Next, 1,026.6 g of sodium hydrogen carbonate aqueous solution (26.6 g of sodium hydrogen carbonate, 1,000.0 g of purified water) was added to the combined organic layers, stirred for 10 minutes and allowed to stand for 10 minutes to separate the layers, followed by discarding the aqueous layer.

200.0 g of sodium sulfate was added to the organic layer, stirred for 10 minutes while maintaining an internal temperature of 20 to 30°C, and then filtered under reduced pressure. The filtrate was washed with 270.6 g of ethyl acetate, the pH of the filtrate was adjusted to 6.0 to 6.5 using 1.0M hydrochloric acid ethyl acetate solution, and then the filtrate was concentrated under reduced pressure at 38°C to 42°C.

### (Additional step - preparation of acid addition salt)

90.2 g of ethyl acetate was added to the concentrated residue, and stirred until a relatively uniform state. 460.5 g of 1.0M hydrochloric acid ethyl acetate solution was gradually added at an internal temperature in the range of 0°C to 5°C, and stirred at 18°C to 22°C for 1 hour to crystallize the compound represented by Chemical Formula 1-1. The resulting crystals were filtered under reduced pressure, and the filtrate was washed with 90.2 g of ethyl acetate. The filtrate was put in a dryer, and vacuum-dried at 40°C to 50°C for 12 hours or more to finally obtain 95.7 g of the compound represented by Chemical Formula 1-1 (yield: 84.7%).
¹H-NMR (500 MHz, MeOD): 7.69(s, 1H), 7.58-7.53(m, 1H), 7.45(t, 1H), 7.30(d, 1H), 7.20-7.15(m, 2H), 7.02-6.94(m, 2H), 4.07(d, 2H), 3.46(s, 3H), 2.71(s, 3H)

### Comparative Example

Comparative Example was conducted in accordance with the following Reaction Scheme.

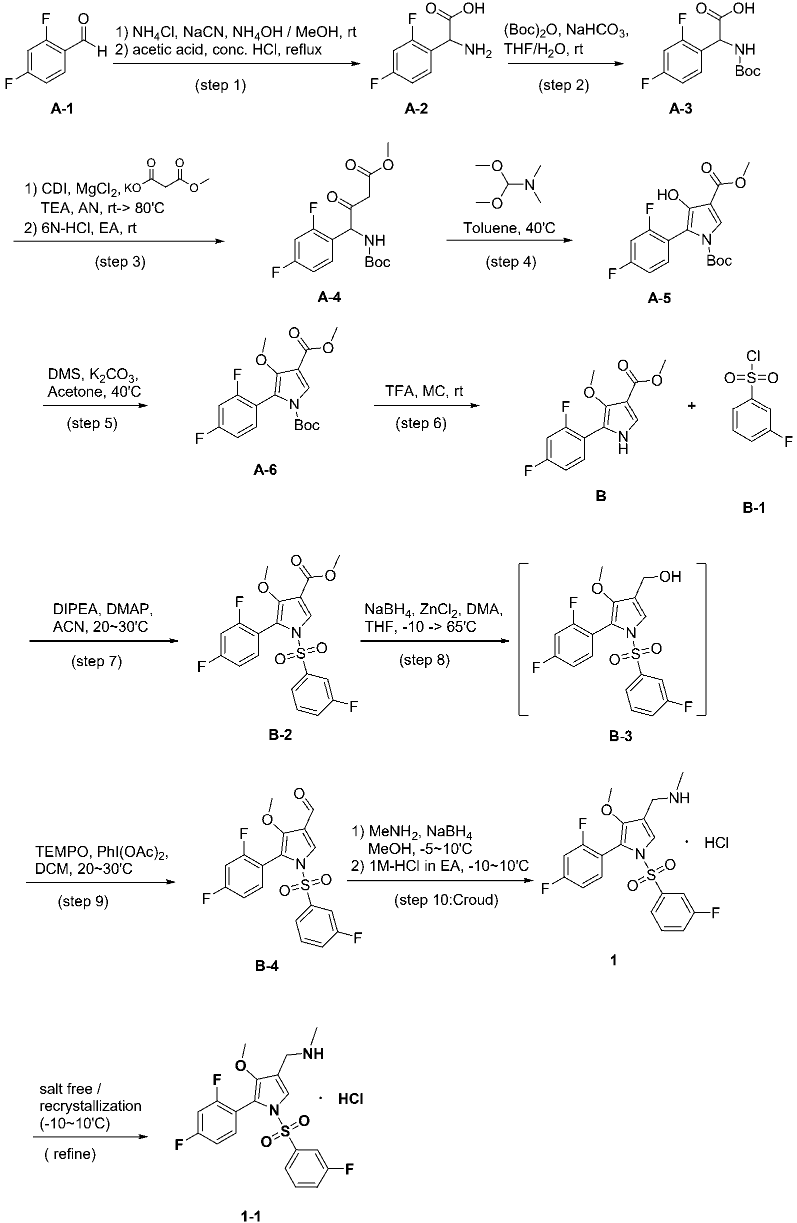

### (Step 1)

35.8 g of ammonium chloride and 26.9 g of sodium cyanide were added to a flask, 716.0 mL of ammonium hydroxide (25 to 28%) was added, and stirred for 10 minutes. The mixture was cooled to 0°C to 5°C, stirred for 10 minutes, then raised o room temperature, and stirred for 15 minutes. After cooling to 0°C to 5°C, a solution containing 100.0 g of 2,4-difluorobenzaldehyde (Chemical Formula A-1) and 770.0 mL of methanol prepared in another flask was gradually added over 15 to 20 minutes. The temperature was raised to room temperature, and the mixture was stirred for 22 hours to complete the first reaction. The mixture was concentrated under reduced pressure at 50°C, and then 983.0 mL of acetic acid, and conc. 983.0 mL of HCl were added and refluxed for 5 hours at an internal temperature of 100°C to 105°C to complete the second reaction. The solvent was removed by concentration under reduced pressure at 75° C. until a solid precipitated. The purified water was added and then stirred to precipitate crystals. The pH was adjusted to 6.5 using a 5M-NaOH solution at an internal temperature of 25°C or less. Ethanol was added, and stirred at 10°C to 15°C for 1 hour. After filtration under reduced pressure, the filtrate was washed with ethanol. The obtained solid was dried under reduced pressure to obtain 78.4 g of the compound represented by Chemical Formula A-2 (yield: 59.5%).

### (Step 2)

100.0 g of the compound represented by Chemical Formula A-2 prepared in step 1, 1.5 L of THF, and 1.5 L of purified water were added to a flask, and the mixture was stirred at room temperature for 10 minutes. The internal temperature was cooled to 0°C to 5°C, and 134.6 g of sodium hydrogen carbonate and 139.5 g of di-tert-butyl dicarbonate were added. The mixture was stirred at an internal temperature of 20°C to 30°C for 12 hours to complete the reaction, and concentrated under reduced pressure at 45°C. Ethyl acetate was added, and cooled down to an internal temperature of 10°C or less. The pH was adjusted to 2.5 using 6N-HCl. The organic layer was separated, and dried over anhydrous magnesium sulfate, and then concentrated under reduced pressure at 45°C to obtain 151.2 g of the compound represented by Chemical Formula A-3 (yield: 98.5%).
¹H-NMR (500 MHz, CDCl₃): 8.13-8.14 (d, 1H), 7.37-7.42 (m, 1H), 6.82-6.89 (m, 2H), 5.46-5.47 (d, 1H), 1.23 (s, 9H)

### (Step 3)

100.0 g of the compound represented by Chemical Formula A-3 prepared in step 2, 61.9 g of carbonyldiimidazole, and 1.0 L of acetonitrile were added to a flask, and the mixture was stirred at room temperature for 1 hour. 59.8 g of methyl potassium malonate, 36.4 g of anhydrous magnesium chloride, 1.0 L of acetonitrile, and 38.8 g of triethylamine were added to another flask, and the mixture was stirred at 20°C to 30°C for 1 hour. The reaction materials in the two flasks were mixed and refluxed at an external temperature of 80°C for 1 hour to complete the reaction. After cooling to room temperature, purified water was added. The mixture was cooled down to an internal temperature of 5°C to 10°C, and stirred for 1 hour. The obtained solid was filtered under reduced pressure, and then washed with purified water. Since the obtained crystal was a magnesium salt, the salt dissociation process was performed below.

The magnesium salt prepared above, 1.5 L of ethyl acetate, and 1.0 L of purified water were added to a flask, and the mixture was stirred for 10 minutes. The pH was adjusted to 7.0 using 6N-HCl. The organic layer was extracted, dried over anhydrous magnesium sulfate, and then concentrated under reduced pressure at 45°C to obtain 97.3 g of the compound represented by Chemical Formula A-4 (yield: 81.4%).
¹H-NMR (500 MHz, CDCl₃): 7.26-7.30 (m, 1H), 6.85-6.92 (m, 2H), 5.83 (s, 1H), 5.64-5.65 (d, 1H), 3.67 (s, 3H), 3.38-3.52 (dd, 2H), 1.41 (s, 9H)

### (Step 4)

100.0 g of the compound represented by Chemical Formula A-4 prepared in step 3, and 2.0 L of toluene were added to a flask, and the mixture was stirred at room temperature for 10 minutes. 104.1 g of N,N-dimethylformamide dimethyl acetal was added, and stirred at 40°C for 4 hours to complete the reaction. The mixture was concentrated under reduced pressure at 45°C, ethyl acetate and purified water were added to the concentrated residue, and stirred for 10 minutes. The pH was adjusted to 7.0 using 1N-HCl. The organic layer was extracted, and dried over anhydrous magnesium sulfate, and then concentrated under reduced pressure at 45°C to prepare 79.2 g of the compound represented by Chemical Formula 1-5 (yield: 77.0%). Meanwhile, the compound represented by Chemical Formula A-5 was unstable (aerial oxidation occurred), and the following step 5 was performed continuously in an in-situ manner.
¹H-NMR (500 MHz, CDCl₃): 7.73 (s, 1H), 7.48 (s, 1H), 7.38-7.43 (q, 1H), 6.83-6.95 (tt, 2H), 3.90 (s, 3H), 1.39 (s, 9H)

### (Step 5)

100.0 g of the compound represented by Chemical Formula A-5 prepared in step 4 and 1.5 L of acetone were added to a flask, and the mixture was stirred at room temperature for 10 minutes. 78.2 g of potassium carbonate and 42.9 g of dimethyl sulfate were added, and stirred at 40°C for 6 hours to complete the reaction. The mixture was cool to room temperature, and purified water and ethyl acetate were added, and stirred for 10 minutes. The pH was adjusted to 7.0 using 6N-HCl. The organic layer was extracted, dried over anhydrous magnesium sulfate, and concentrated under reduced pressure at 45°C to obtain 90.6 g of the compound represented by Chemicial Formula A-6 (yield: 87.1%). Next, the following step 6 was performed in an in-situ manner without a separate purification step.
¹H-NMR (500 MHz, CDCl₃): 7.87 (s, 1H), 7.31-7.36 (q, 1H), 6.84-6.95 (tt, 2H), 3.86 (s, 3H), 3.68 (s, 3H), 1.38 (s, 9H)

### (Step 6)

100.0 g of the compound represented by Chemical Formula A-6 prepared in step 5 and 500.0 mL of methylene chloride were added to a flask, and the mixture was stirred at room temperature for 10 minutes. 310.4 g of trifluoroacetic acid was added, and stirred at room temperature for 6 hours to complete the reaction. Then, after cooling to 0°C to 5°C, purified water was gradually added at 15°C or less. The pH was adjusted to 7.0 at 15°C or less using a 50.0% NaOH solution. Ethyl acetate was added, and stirred for 10 minutes. The organic layer was extracted, and dried over anhydrous magnesium sulfate. Celite washed with ethyl acetate was placed on a filter, and the organic layer was filtered under reduced pressure, and then concentrated under reduced pressure at 45°C. Ethyl acetate was added to the concentrated residue, and the mixture was stirred and suspended. n-hexane was added, cooled down to an internal temperature of 0°C to 5°C, and stirred for 1 hour. The obtained solid was filtered under reduced pressure, and then the filtrate was washed with n-hexane. The result was dried reduced pressure to obtain 65.5 g of the compound represented by Chemical Formula B (yield: 90.0%).
¹H-NMR (500 MHz, CDCl₃): 8.78 (s, 1H), 8.12 (m, 1H), 7.30 (d, 1H), 6.95 (t, 1H), 6.88 (t, 1H), 3.87 (s, 3H), 3.85 (s, 3H)

### (Step 7)

100.0 g of methyl 5-(2,4-difluorophenyl)-4-methoxy-1H-pyrrole-3-carboxylate (Chemical Formula B), 9.2 g of 4-(dimethylamino)-pyridine, and 393.0 g of acetonitrile were added to a flask, and the mixture was stirred at room temperature for 10 minutes. The internal temperature of the flask was cooled to 5°C to 10°C, 80.1 g of 3-fluorobenzenesulfonyl chloride (Chemical Formula B-1) and 53.2 g of N,N-diisopropylethylamine were added, and stirred in a temperature range of 20°C to 30°C for 2 hours to complete the reaction.

Next, 500.0 g of purified water and 451.0 g of ethyl acetic acid were added, stirred for 10 minutes and allowed to stand for 10 minutes, and then the aqueous layer was discarded.

Then, 500.0 g of purified water was added to the organic layer, and 1N-hydrochloric acid aqueous solution was gradually added within a temperature range of 20°C to 30°C, and the pH was adjusted to 3.5 to 5.0. The mixture was stirred for 10 minutes, allowed to stand for 10 minutes, and the aqueous layer generated here was discarded.

Next, the organic layer was concentrated under reduced pressure at 50°C to 55°C, 158.4 g of methanol was added at an internal temperature of 20°C to 30°C, and stirred for 10 minutes. Then, the organic layer was concentrated under reduced pressure at 50°C to 55°C, 396.0 g of methanol was added at an internal temperature of 20°C to 30°C, and then stirred for 1 hour. While maintaining the internal temperature at 20°C to 30°C, 300.0 g of purified water was added for 20 minutes, and stirred for 1 hour. The resulting crystals were filtered under reduced pressure, and the filtrate was washed with 200.0 g of purified water.

The washed filtrate was put in a dryer, and dacuum-dried at a temperature of 40°C to 45°C for 12 hours or more to obtain 154.4 g of the compound represented by Chemical Formula B-2 (yield: 97.0%).
¹H-NMR (500 MHz, MeOD): 7.98 (s, 1H), 7.43-7.39 (m, 1H), 7.30 (t, 1H), 7.23 (d, 1H), 7.15 (q, 1H), 7.67 (q, 1H), 6.91 (t, 1H), 6.77 (t, 1H), 3.87 (s, 3H), 3.61 (s, 3H)

### (Step 8)

100.0 g of the compound represented by Chemical Formula B-2 obtained in step 1 and 444.5 g of tetrahydrofuran were added to a new flask, and the mixture was stirred at 20°C to 30°C for 10 minutes, and cooled down to an internal temperature of -10°C to -5°C. 32.1 g of zinc chloride was added to the resulting reaction solution for 5 minutes, and stirred for 10 minutes. 28.5 g of N,N-dimethylaniline was added, and then stirred.

Then, while keeping the internal temperature at the temperature range of -10°C to 0°C, 8.9 g of sodium borohydride was divided into three and added for 5 minutes, and then stirring was repeated three times for 10 minutes.

Next, the mixture was stirred at an internal temperature of 60°C to 65°C for 20 hours to complete the reaction, and then cooled down to an internal temperature of 0°C to 5°C. In this reaction, a compound represented by Chemical Formula B-3 was produced.

Subsequently, 200.0 g of purified water was gradually added at an internal temperature in the range of 0°C to 15°C, and 451.0 g of ethyl acetate was added at an internal temperature in the range of 20°C to 30°C. Then, 87.2 g of 6N-hydrochloric acid aqueous solution was added, stirred for 30 minutes and allowed to stand for 10 minutes (however, the internal temperature was maintained at 20°C to 30°C). The layers were separated and the aqueous layer was discarded. Next, the organic layer was washed using 300.0 g of purified water and 10.9 g of 6N-hydrochloric acid aqueous solution (however, repeated twice while maintaining the internal temperature at 20°C to 30°C). The layers were separated and the aqueous layer was discarded. 50.0 g of magnesium sulfate was added to the organic layer, stirred for 10 minutes, and then filtered under reduced pressure. The filtrate was concentrated under reduced pressure at 50°C to 55°C. Then, 265.3 g of methylene chloride was added, and stirred for 10 minutes, the mixture was concentrated under reduced pressure at 50°C to 55°C.

### (Step 9)

6.9 g of (2,2,6,6-tetramethylpiperidin-1-yl)oxyl, 86.1 g of (diacetoxyiodine)benzene, and 1,171.1 g of dichloromethane were added to the concentrated residue according to step 2, and the mixture was stirred at an internal temperature of 20°C to 30°C for 2 hours to complete the reaction, and then 882.8 g of purified water was added. Then, 679.7 g of saturated sodium hydrogen carbonate aqueous solution (61.8 g of sodium hydrogen carbonate, 617.9 g of purified water) was gradually added, then stirred for 10 minutes, allowed to stand for 10 minutes to separate the layers. Thereafter, the aqueous layer was discarded. 17.7 g of magnesium sulfate was added to the organic layer, stirred for 10 minutes, and then filtered under reduced pressure.

Subsequently, after concentration under reduced pressure at 38°C to 42°C, 513.6 g of ethanol aqueous solution (390.0 g of ethanol, 123.6 g of purified water) was added thereto, and stirred at an internal temperature of 20°C to 30°C for 1 hour to crystallize.

The resulting crystals were filtered under reduced pressure, and the filtrate was washed with 174.3 g of ethanol aqueous solution (132.3 g of ethanol, 41.9 g of purified water). The washed filtrate was put in a dryer, and vacuum-dried at a temperature of 40°C to 45°C for 12 hours or more to obtain 83.9 g of the compound represented by Chemical Formula B-4 (yield: 90.0%).
¹H-NMR (500 MHz, MeOD): 9.89 (s, 1H), 7.99 (s, 1H), 7.45-7.41 (m, 1H), 7.33 (s, 1H), 7.25 (d, 1H), 7.18 (q, 1H), 7.05 (s, 1H), 6.92 (t, 1H), 6.77 (t, 1H), 3.63 (s, 3H)

### (Step 10)

100.0 g of the compound represented by Chemical Formula B-4 obtained in step 3, 396.0 g of methanol, and 48.5 g of methylamine (9.8M in methanol) were added to a new flask, the internal temperature was adjusted to 20°C to 30°C, and the mixture was stirred for 30 minutes.

Then, the internal temperature was cooled to -5°C to 0°C, 4.8 g of sodium borohydride was added in divided portions while maintaining the temperature range of - 5°C to 10°C, and stirred at -5°C to 10°C for 30 minutes to complete the reaction.

After completion of the reaction, 1,000 g of purified water was gradually added while maintaining the internal temperature at 10°C to 15°C, and hen 902.0 g of ethyl acetate was added. Then, the internal temperature was maintained at 10°C to 15°C, and the pH was adjusted to 6.7 to 7.3 using 6N-hydrochloric acid aqueous solution.

Then, the mixture was stirred for 10 minutes, allowed to stand for 30 minutes to separate the layers, and the organic layer was stored. 451.0 g of ethyl acetate was added to the resulting aqueous layer, stirred for 10 minutes, and then allowed to stand for 10 minutes. Then, layer separation was performed, the organic layer was combined with the previously obtained organic layer, and the same re-extraction process was performed once again.

Next, 600.0 g of sodium chloride aqueous solution (100.0 g of sodium chloride, 500.0 g of purified water) was added to the combined organic layers, stirred for 10 minutes, and allowed to stand for 10 minutes to separate layers, and the aqueous layer was discarded.

100.0 g of magnesium sulfate was added to the organic layer, stirred for 10 minutes while maintaining an internal temperature of 10°C to 15°C, and then filtered under reduced pressure. The filtrate was washed with 270.6 g of ethyl acetate, and then the filtrate was concentrated under reduced pressure at 38°C to 42°C.

90.2 g of ethyl acetate was added to the concentrated residue, stirred until a relatively uniform state, and 460.5 g of 1.0M hydrochloric acid ethyl acetate solution was gradually added at an internal temperature in the range of -5°C to 5°C. The mixture was then stirred at 0°C to 5°C for 12 hours to crystallize the compound represented by Chemical Formula 1.

### (Refine step)

Next, the crystals obtained in step 10 were filtered under reduced pressure, and the filtrate was washed with 90.2 g of ethyl acetate. The filtrate and 815.4 g of ethyl acetate were added to a new flask, and cooled down to an internal temperature of 0°C to 15°C, and then stirred for 10 minutes. Then, 976.3 g of sodium hydrogen carbonate aqueous solution (72.3 g of sodium hydrogen carbonate, 904.0 g of purified water) was added at an internal temperature of 10°C to 15°C, stirred for 10 minutes, and allowed to stand for 30 minutes to separate the layers, and the organic layer was stored.

407.7 g of ethyl acetate was added to the resulting aqueous layer, stirred for 10 minutes, and then allowed to stand for 10 minutes to separate the layers. The organic layer was combined with the organic layer obtained earlier, and the aqueous layer was reextracted once again using the same process and combined with the organic layer.

90.4 g of magnesium sulfate was added to the organic layer, stirred for 10 minutes at an internal temperature of 10°C to 15°C, and then filtered under reduced pressure. The filtrate was washed with 244.6 g of ethyl acetate, and then the filtrate was concentrated under reduced pressure at 38°C to 42°C.

81.5 g of ethyl acetate was added to the concentrated residue, stirred, and the mixture was stirred. 368.4 g of 1.0 M hydrochloric acid ethyl acetate solution was gradually added at an internal temperature in the range of -5°C to 5°C, and stirred at 0°C to 5°C for 12 hours to recrystallize.

The resulting crystals were filtered under reduced pressure, the filtrate was washed with 81.5 g of ethyl acetate, and then the filtrate was put in a dryer, and vacuum-dried at 20°C to 30°C for 12 hours, and further dried for 6 hours by heating to 38°C to 42°C to finally obtain 90.7 g of the compound represented by Chemical Formula 1-1 (yield: 80.2%).
¹H-NMR (500 MHz, MeOD): 7.69(s, 1H), 7.58-7.53(m, 1H), 7.45(t, 1H), 7.30(d, 1H), 7.20-7.15(m, 2H), 7.02-6.94(m, 2H), 4.07(d, 2H), 3.46(s, 3H), 2.71(s, 3H)

### Comparison of Examples and Comparative Examples

The yield, quality, etc. of the 4-methoxy pyrrole derivatives obtained according to the respective manufacturing methods of Examples and Comparative Examples were evaluated as follows, and are shown in Table 1 below.

The yield of 4-methoxy pyrrole derivatives: It was calculated by substituting the weight of 4-methoxy pyrrole derivatives (Chemical Formula 1) recovered after completion of the reaction and the weight of methyl 5-(2,4-difluorophenyl)-4-methoxy-1H-pyrrole-3-carboxylate (Chemical Formula 2) before the reaction into the following Equation 1.

### [Equation 1]

The yield of 4-methoxy pyrrole derivatives (%)=100%* {number of moles of 4-methoxy pyrrole derivatives (Chemical Formula 1) recovered after completion of reaction} / {number of moles of methyl 5-(2,4-difluorophenyl)-4-methoxy-1H-pyrrole-3-carboxylate (Chemical Formula 2) before reaction}

The purity of 4-methoxy pyrrole derivatives and the content of related substance B: The purity of 4-methoxy pyrrole derivatives (Chemical Formula 1) recovered after completion of the reaction and the content of related substance B were measured using high performance liquid chromatography (HPLC, manufacturer: Waters, e2695 system).

Herein, the related substance B is 1-(5-(2,4-difluorophenyl)-4-methoxy-1H-pyrrol-3-yl)-N-methylmethanamine.

**[Table 1]**

| | Comparative Example | Example |
|---|---|---|
| Number of process step | 10 | 6 |
| Yield | 20.2% | 30.1% |
| Quality | Purithy 99.5% or more | Purity 99.7% or more |
| | Related substance B 0.05% or less | Related substance B 0.05% or less |

Referring to Table 1, it was confirmed that Examples not only improved the process efficiency compared to Comparative Examples, but also improved the total yield by about 1.5 times compared to Comparative Examples.

In addition, when applying the manufacturing methods of Examples and Comparative Examples to industrial production, the manufacturing methods of the Comparative Examples required 17 days to produce the final material, while Examples required 9 days. That is, since the manufacturing time of Examples was reduced by half compared to Comparative Examples, it is expected that the production efficiency will be doubled.

Furthermore, according to Examples, it is expected that industrial mass production will be possible by ensuring a stepwise crystallization process.

Specifically, in Examples and Comparative Examples, the same final material (i.e., the 4-methoxy pyrrole derivative represented by Chemical Formula 1) was prepared by using the same starting material (i.e., the compound represented by Chemical Formula 1-1). Further, since Examples do not use dimethyl sulfate, which is a genotoxic substance used in Comparative Examples, it is possible to reduce the safety of workers and the potential danger of pharmaceuticals.

## Claims

1. A manufacturing method for 4-methoxypyrrole derivatives, the method comprising the steps of:
1) reacting a compound represented by the following Chemical Formula 1-1 with p-toluenesulfinic acid and formamide in the presence of an acid catalyst to prepare a compound represented by the following Chemical Formula 1-2;
2) reacting the compound represented by the following Chemical Formula 1-2 with a dehydrating reagent to prepare a compound represented by the following Chemical Formula 1-3;
3) reacting the compound represented by the following Chemical Formula 1-3 with a cyclization reagent to prepare a compound represented by the following Chemical Formula 1-4;
4) reacting the compound represented by the following Chemical Formula 1-4 with 3-fluorobenzenesulfonyl chloride to prepare a compound represented by the following Chemical Formula 1-5;
5) reacting the compound represented by the following Chemical Formula 1-5 with sodium hydride to prepare a compound represented by the following Chemical Formula 1-6; and
6) reacting the compound represented by the following Chemical Formula 1-6 with methylamine to form an intermediate, and then adding a reducing agent to convert the intermediate to a compound represented by the following Chemical Formula 1:

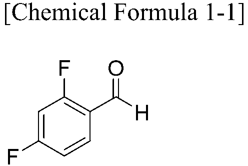

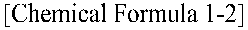

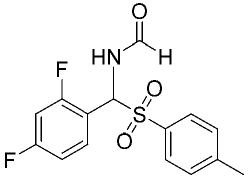

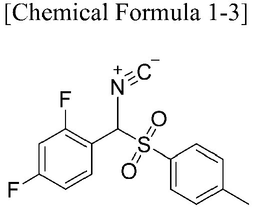

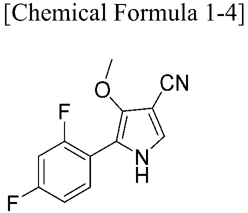

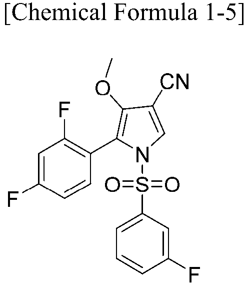

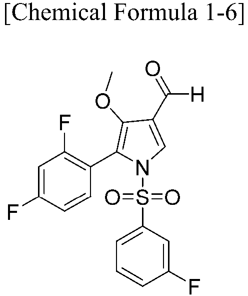

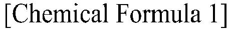

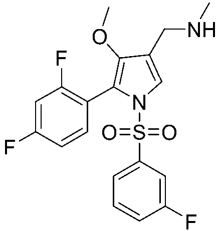

2. The manufacturing method according to claim 1, wherein the step 1 is performed in the presence of chlorotrimethylsilane (TMSCl), camphorsulfonic acid, trifluoromethanesulfonic acid, trifluoroacetic acid, benzoic acid, nitrobenzoic acid, hydrochloric acid, calcium chloride chloride or a mixture of two or more thereof as an acid catalyst.

3. The manufacturing method according to claim 1, wherein a molar ratio between the compound represented by Chemical Formula 1-1 and the acid catalyst is 20:1 to 1:10.

4. The manufacturing method according to claim 1, wherein a molar ratio between the compound represented by Chemical Formula 1-1 and formamide is 10:1 to 1:10.

5. The manufacturing method according to claim 1, wherein p-toluenesulfinic acid in the step 1 is a concentrate concentrated to 10 mol% or more.

6. The manufacturing method according to claim 1, wherein the reaction temperature in the step 1 is 0°C to 80°C.

7. The manufacturing method according to claim 1, wherein the dehydrating reagent of the step 2 comprises phosphoryl chloride, trichloromethyl chloroformate, bis(trichloromethyl)carbonate, triphenylphosphine, thionyl chloride, p-toluenesulfonic acid or a mixture of two or more thereof.

8. The manufacturing method according to claim 1, wherein a molar ratio between the compound represented by Chemical Formula 1-2 and the dehydrating reagent is 20:1 to 1:10.

9. The manufacturing method according to claim 1, wherein the step 2 is performed in the presence of a base, and the molar ratio between the compound represented by Chemical Formula 1-2 and the base in the step 2 is 20:1 to 1:20.

10. The manufacturing method according to claim 1, wherein the reaction temperature in the step 2 is -20°C to 20°C.

11. The manufacturing method according to claim 1, wherein the cyclization reagent of the step 3 includes ethyl (Z)-2-cyano-3-methoxyacrylate, methyl (Z)-2-cyano-3-methoxyacrylate, propyl (Z)-2-cyano-3-methoxyacrylate, or a mixture of two or more thereof.

12. The manufacturing method according to claim 1, wherein in the step 3, the molar ratio between the compound represented by Chemical Formula 1-4 and the cyclization reagent is 10:1 to 1:10.

13. The manufacturing method according to claim 1, wherein the step 3 is performed in the presence of a base, and the molar ratio between the compound represented by Chemical Formula 1-3 and the base in the step 3 is 20:1 to 1:10.

14. The manufacturing method according to claim 1, wherein the reaction temperature of the step 3 is 50 to 150°C.

15. The manufacturing method according to claim 1, further comprising dissolving the compound represented by Chemical Formula 1-4 in an organic solvent together with a catalyst to prepare a composition containing the compound represented by Chemical Formula 1-4, before the step 4.

16. The manufacturing method according to claim 15, wherein the catalyst comprises 4-dimethylaminopyridine, 4-pyrrolidinylpyridine, pyridine, 1,5,7-triazabicyclo[4.4.0]dec-5-ene, 1,8-diazabicyclo [5.4.0]undec-7-ene, 7-methyl-1,5,7-triazabicyclo[4.4.0]dec-5-ene, or a mixture thereof.

17. The manufacturing method according to claim 15, wherein in the step 4, the molar ratio between the compound represented by Chemical Formula 1-4 and the catalyst is 100:1 to 1:10.

18. The manufacturing method according to claim 1, wherein in the step 4, the molar ratio between the compound represented by Chemical Formula 1-4 and 3-fluorobenzenesulfonyl chloride is 10:1 to 1:10.

19. The manufacturing method according to claim 1, wherein the step 4 is performed in the presence of a base, and the molar ratio between the compound represented by Chemical Formula 1-4 and the base in the step 4 is 10:1 to 1:10.

20. The manufacturing method according to claim 1, wherein the reaction temperature of the step 4 is 0 to 60°C.

21. The manufacturing method according to claim 1, wherein the sodium hydride of the step 5 is a dispersion in mineral oil.

22. The manufacturing method according to claim 1, wherein in the step 5, the molar ratio between the compound represented by Chemical Formula 1-5 and sodium hydride is 20:1 to 1:10.

23. The manufacturing method according to claim 1, wherein the step 5 is performed in the presence of zinc halide.

24. The manufacturing method according to claim 23, wherein the zinc halide comprises zinc chloride, zinc bromide, zinc iodide, or a mixture of two or more thereof.

25. The manufacturing method according to claim 23, wherein in the step 5, the molar ratio between the compound represented by Chemical Formula 1-5 and zinc halide is 10:1 to 1:10.

26. The manufacturing method according to claim 1, wherein the reaction temperature of the step 5 is 20 to 80°C.

27. The manufacturing method according to claim 1, wherein in the step 6, the reaction temperature of the compound represented by Chemical Formula 1-6 and methyl amine is 10 to 30°C.

28. The manufacturing method according to claim 1, wherein the reducing agent in the step 6 comprises sodium borohydride, sodium cyanoborohydride, sodium triacetoxyborohydride, or a mixture of two or more thereof.

29. The manufacturing method according to claim 1, wherein in the step 6, the reaction temperature of the reducing agent and the intermediate is -5 to 10°C.

30. The manufacturing method according to claim 1, wherein in the step 6, the molar ratio between the compound represented by Chemical Formula 1-6 and the methylamine is 10:1 to 1:10, and the molar ratio between the compound represented by Chemical Formula 1-6 and the reducing agent is 10:1 to 1:10.

31. The manufacturing method according to claim 1, further comprising washing the reaction product of the step 6 with an aqueous base solution to adjust the pH to 6.0 to 6.5.

32. The manufacturing method according to claim 1, further comprising an additional step of adding an acid to the compound represented by Chemical Formula 1 to prepare an acid salt represented by the following Chemical Formula 1-1, after the step 6:

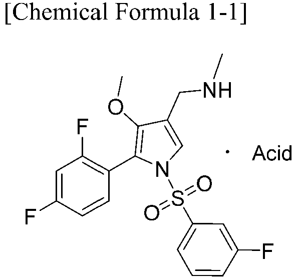

33. The manufacturing method according to claim 32, wherein the additional step comprises supplying an organic solvent to the compound represented by Chemical Formula 1 and then supplying an acid or a mixed solution thereof with an organic solvent to crystallize the acid salt represented by Chemical Formula 1-1.
